# DEMANDE DE BREVET EUROPEEN

(11) **EP 1 219 247 A1**
(43) Date de publication de la demande: **03.07.2002**
(21) Numéro de dépôt: 00204750.4
(22) Date de dépôt: 26.12.2000
(51) Int. Cl.: A61B 17/122

(54) **Dispositif de clampage pour structure anatomique**

(71) Demandeur: Cardio Life Research S.P.R.L., 1050 Bruxelles (BE)
(72) Inventeur: De Cannière, Bernard, 1050 Bruxelles (BE)
(74) Mandataire: Vandeberg, Marie-Paule L.G.

(57) **Abrégé**

Un dispositif de clampage chirurgical pour structures anatomiques caves, telles que, essentiellement, les vaisseaux sanguins, mais aussi trachées, intestins, etc.

Ce dispositif comprend un guide (20) flexible; deux mâchoires (30) formées chacune d'un membre allongé percé d'un canal longitudinal (32) et présentant une extrémité distale et une extrémité proximale, ces mâchoires (30) étant enfilables sur le guide (20) avec leur extrémité proximale tournée vers chacune des extrémités du guide; un mandrin (34) enfilable sur les deux extrémités du guide (20) et apte à rapprocher l'un de l'autre les axes des deux mâchoires (30) et un dispositif d'actionnement du pinçage .

Le dispositif de l'invention s'applique plus particulièrement au clampage de l'aorte.

## Description

L'invention concerne les dispositifs de clampage pour structures anatomiques caves tubulaires, telles que, essentiellement, les vaisseaux sanguins, mais aussi trachées, intestins, etc.

### Arrière-plan technologique de l'invention

La chirurgie cardiaque requiert le plus souvent un arrêt cardiaque afin d'obtenir un site opératoire immobile et exsangue permettant un geste chirurgical précis et délicat. Ceci nécessite : 1) le recours à la circulation extra-corporelle (CEC) afin de perfuser les organes systémiques (cerveau, foie, reins etc.) avec du sang oxygéné pendant la période ou le coeur est arrêté.
2) Le clampage de l'aorte qui consiste à obturer le vaisseau par une pince externe qui est interposée entre la canule artérielle permettant la circulation extra-corporelle et l'orifice des artères coronaires. Cette manoeuvre isole la circulation coronaire du flux sanguin apporté par la CEC et donc permet d'arrêter le coeur.
3) La cardioplégie : injection d'une solution dans le réseau des artères coronaires pour protéger le coeur lui-même pendant la période d'arrêt.

La mise en place de la circulation extra-corporelle (CEC), le clampage et la cardioplégie nécessitent classiquement la découpe et l'écartement du sternum (sternotomie). La sternotomie est une voie d'abord chirurgicale délabrante et porteuse de complications post opératoires fréquentes pour les patients.

Par ailleurs, le clampage de l'aorte est une opération considérée comme délicate et risquée du fait, notamment, de la proximité de l'artère pulmonaire, dont la texture est connue comme extrêmement fragile.

En outre, le pincement de l'artère à l'aide d'un clamp classique est source d'embolisation de matériel athéromateux qui tapisse dans la plupart des cas la paroi interne du vaisseau.

Depuis plusieurs années, la chirurgie cardiaque développe des techniques alternatives visant à procurer une moindre agression pour les patients. L'éviction de la sternotomie est une de ces approches. Dans ce cas, l'intervention est conduite par des mini-incisions permettant l'introduction d'instruments endoscopiques.

Dans ce type d'intervention, quand le coeur doit être arrêté, un ballon gonflable est introduit à l'intérieur de l'artère aorte, à la sortie du coeur, sous contrôle échographique ou fluoroscopique, pour boucher l'aorte de l'intérieur du vaisseau.

Ce système présente de nombreux inconvénients dont, notamment, son coût, qui en limitent l'utilisation à un nombre de cas marginaux. En outre, on déloge également, par cette technique, des particules entraînées par le flux sanguin.

Aucune alternative ne permet actuellement d'interrompre la circulation du sang dans l'aorte sans ouvrir le thorax.

On a donc recherché une solution alternative permettant à un grand nombre de patients de subir en toute sécurité une intervention cardiaque aussi peu invasive que possible.
L'objet de l'invention est un clamp chirurgical qui comprend
- un guide flexible;
- deux mâchoires formées chacune d'un membre allongé percé d'un canal longitudinal et présentant une extrémité distale et une extrémité proximale, les dites mâchoires étant enfilables sur le guide avec leur extrémité proximale tournée vers chacune des extrémités du guide;
- un mandrin enfilable sur les deux extrémités du guide et sur les mâchoires et apte à rapprocher l'un de l'autre les axes des deux mâchoires;
- un organe d' actionnement du pinçage.

La fermeture des mâchoires est assurée de préférence par une tige de commande enfilée sur les deux extrémités du guide en amont du mandrin.

Avantageusement, une gaine intermédiaire est mise en place à l'aide du guide. Cette gaine souple peut être positionnée dans le thorax en regard d'une structure vasculaire encerclée par le guide.

Les mors présentent de préférence une section malléable de côté de leur extrémité proximale.

Suivant un mode de réalisation avantageux, le mandrin comprend un seul canal longitudinal, l'extrémité distale de cette cavité étant profilée de façon à entraîner le rapprochement des axes des deux mâchoires par un déplacement relatif.

Suivant un autre mode de réalisation avantageux le mandrin comprend deux canaux longitudinaux, chacune des deux mâchoires étant enfilées dans un des deux canaux.

Les mâchoires sont pourvues de préférence, près de leur extrémité distale, de mors souples.

Suivant un mode de réalisation avantageux, les mâchoires sont serrées par un dispositif de solidarisation à articulation unique ou multiples.

L'avantage du clamp de l'invention est que l'on obtient un clampage efficace et puissant par une incision minime, de l'ordre du centimètre, rendant par là l'intervention extrêmement peu invasive.

Le dispositif de l'invention s'applique de façon particulièrement avantageuse au clampage de l'aorte grâce à l'utilisation de l'espace anatomique du sinus transverse de Theile comme guide naturel.

Un autre avantage est que le risque d'abîmer un organe adjacent est réduit à un minimum. En particulier dans le cas d'interventions cardiaques, le risque de déchirer l'artère pulmonaire devient pratiquement négligeable, puisqu'il n'est plus nécessaire de pratiquer la dissection du plan virtuel entre les deux vaisseaux.

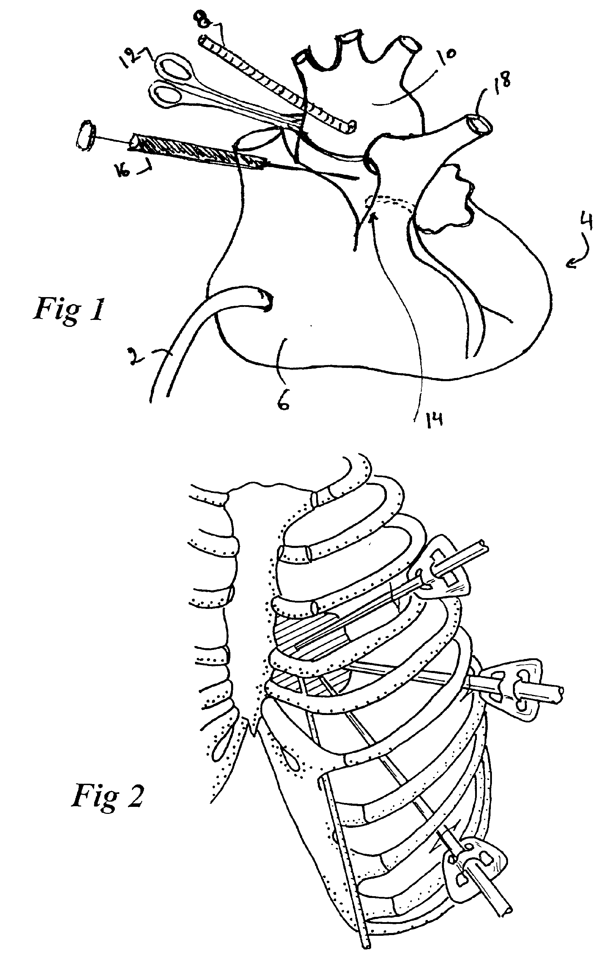

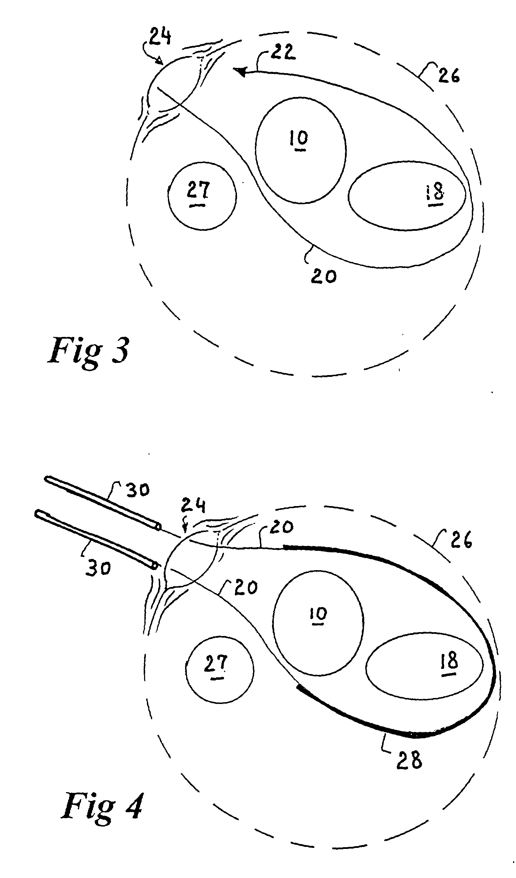

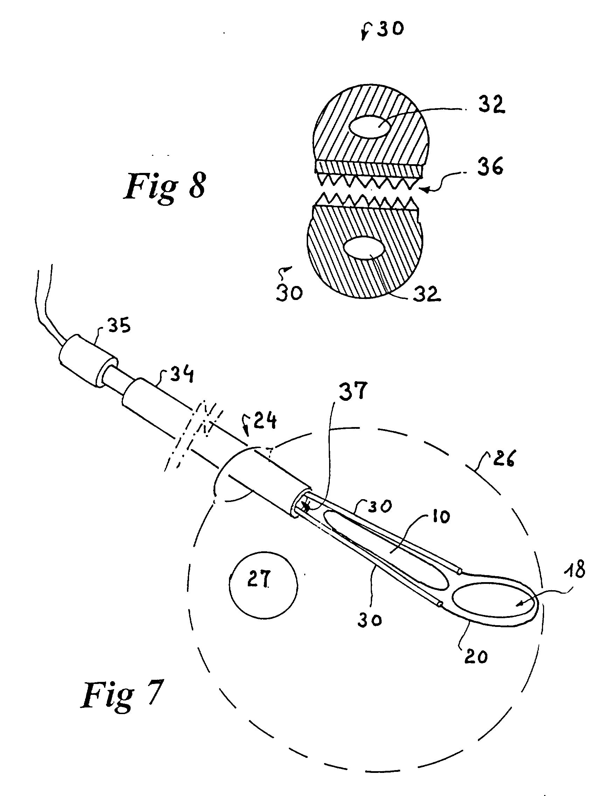

Le clamp de l'invention peut être utilisé aussi bien pour des structures vasculaires intra et extra thoraciques qu'à d'autres structures anatomiques dont notamment les intestins. Il peut également être utilisé comme davier pour manipuler des os.
D'autres particularités et avantages de l'invention ressortiront de la description ci-après de modes de réalisation particuliers de l'invention, référence étant faite aux dessins annexés dans lesquels :
○ La Fig. 1 est une vue schématique en perspective du clampage d'un coeur humain dans le cas d'une opération avec sternotomie.
○ La Fig. 2 est une vue avec arrachement d'une opération du coeur avec mini-incision intercostale.
○ Les Fig. 3 à 7 sont des vues schématiques du principe de clampage du dispositif de l'invention.
○ La Fig. 8 est une vue en coupe d'une paire de mors du clamp de l'invention.
La Fig. 1 montre les différentes opérations préparatoires à une intervention cardiaque classique, afin d'obtenir un champ opératoire exsangue et immobile .

Le sang veineux (pauvre en oxygène) est dérivé par une canule 2 lors de son entrée dans le coeur 4 par l'oreillette droite 6 vers une machine coeur-poumons (non représentée) qui le réoxygène et le débarrasse de son CO₂. Le sang artificiellement oxygéné est ensuite réinjecté par une deuxième canule 8 à hauteur de l'aorte 10 dans le circuit artériel du patient, court-circuitant ainsi le coeur 4 et la circulation pulmonaire afin de permettre le geste opératoire intra ou extra-cardiaque.

Le coeur 4 peut alors être arrêté pour obtenir un champ opératoire exsangue et immobile.

L'arrêt cardiaque est classiquement obtenu par deux manoeuvres concomitantes :
- Le clampage aortique;
- L'injection d'une solution de cardioplégie dans la circulation coronaire.

Le clampage de l'aorte 10 consiste à obturer le vaisseau par une pince 12 externe qui est interposée entre la canule artérielle 8 de la circulation extra-corporelle et l'orifice des artères coronaires 14. Cette manoeuvre isole la circulation coronaire du flux sanguin engendré par la CEC.

Une solution de cardioplégie peut dès lors être injectée par un organe d'injection 16 dans la circulation coronaire pour "paralyser" le coeur 4 dans le but de permettre au chirurgien un geste plus précis qu'il ne le serait sur des structures anatomiques en mouvement.

La Fig. 2 montre une autre approche connue, dans laquelle une intervention cardiaque est menée via une ou plusieurs incisions de l'ordre du centimètre permettant l'introduction d'instruments endoscopiques.

Le problème qui se pose dans ce cas est d'interrompre de façon sûre la circulation du sang dans l'aorte en évitant les inconvénients inhérents à l'introduction d'un ballonnet.

Comme il a été dit plus haut, il est impossible d'avoir recours à une pince classique dont la dimension des mâchoires et leur course sont hors de proportion avec celles des incisions intercostales pratiquées.

L'avantage majeur du système selon l'invention est de permettre le clampage sans ouverture du thorax au prix, qui plus est, d'un risque moindre de traumatisme de l'artère pulmonaire et d'embolie.

Le dispositif de clampage de l'invention et ses différents composants seront décrits en se référant à la succession des Fig. 3 à 7.

Un guide 20 souple, éventuellement muni d'une extrémité 22 dirigeable, est introduit dans le thorax par voie gauche ou droite dans un trou de port, puis par une incision 24 dans le péricarde dans le sinus transverse de Theile, espace anatomique virtuel naturel 26 recouvert de séreuse péricardique. Il suffit de pousser le guide souple 20 pour qu'il réapparaisse en encerclant les vaisseaux à clamper. En l'occurrence, il s'agit des gros vaisseaux de la base du coeur, c'est-à-dire l'aorte 10 et l'artère pulmonaire 18. Le guide permet d'éviter d'autres organes (tels qu'ici, la veine cave supérieure 27). L'extrémité 22 de ce guide 20 est alors recapturée par une contre incision si nécessaire et ressorti par le même trou de port (Figure 3).

Suivant les caractéristiques mécaniques du guide et la nature des vaisseaux à enserrer, une gaine intermédiaire 28 (Figure 4) est enfilée sur le guide souple 20. Suivant une autre technique, cette gaine intermédiaire 28 est accrochée à une des extrémités du guide et hâlée en place.

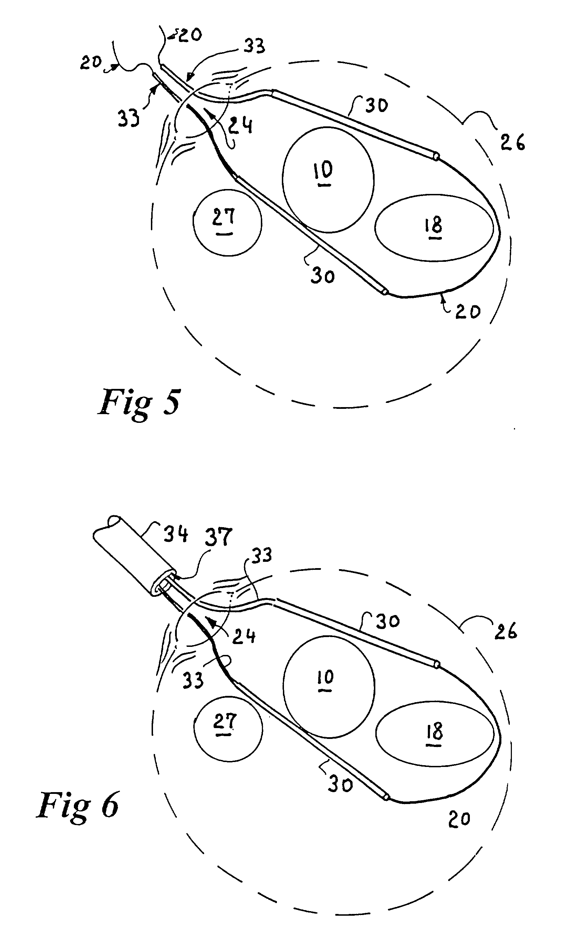

L'opérateur enfile ensuite (Fig. 4) sur chacune des extrémités du guide 20 (ou de la gaine intermédiaire 28)une mâchoire 30 formée d'un membre allongé percé d'un canal longitudinal 32 (visible à la Fig. 8), ces mâchoires 30 sont déplacées de façon à ce que leurs extrémités distales viennent se placer de part et d'autre au vaisseau à enserrer (en l'occurrence, l'aorte 10). Les mâchoires 30 représentées aux Fig. 5 et 6 diffèrent des mâchoires 30 de la Fig. 4 en ce qu'elles comprennent chacune, à leur extrémité proximale, une partie malléable 33.

Les mâchoires 30 étant en place, l'opérateur enfile sur les deux extrémités du guide souple 20 un mandrin 34 creux (Fig. 6) et fait remonter ce mandrin 34 vers l'extrémité proximale des mâchoires 30 (Fig. 7). Les mâchoires 30 s'alignent alors chacune suivant l'axe du mandrin 34 et se rapprochent l'une de l'autre, entraînant un pincement délicat et progressif de l'aorte 10.

Le dispositif entraînant le pinçage peut être agencé de différentes façons :

Dans sa conception la plus simple, la partie proximale des mâchoires comprend une surface conique qui coopère avec l'embouchure du canal longitudinal 37 du mandrin 34, entraînant un alignement des mâchoires. Ce canal 37 peut être simple ou double. Les mâchoires peuvent également être munies de cannelures coopérant avec un pas de vis ménagé à la surface interne du mandrin 34.

Suivant un autre agencement (Figure 7), une pièce 35 mobile par rapport au mandrin 34 est montée sur les deux extrémités proximales des mâchoires 34. Par déplacement relatif de cette pièce 35 avec le mandrin 34, notamment par l'intermédiaire d'une partie filetée, on obtient un serrage ou un desserrage des mâchoires 30.

La Fig. 8 montre, en coupe, la partie distale des mâchoires 30. Dans ce mode de réalisation, celles-ci sont pourvues de mors souples 36 de façon à répartir la pression sur l'organe saisi. On distingue sur cette coupe le canal longitudinal 32 permettant de faire passer le guide 20.

## Revendications

1. Clamp chirurgical **caractérisé en ce qu'**il comprend un
- guide (20) flexible;
- deux mâchoires (30) formées chacune d'un membre allongé percé d'un canal longitudinal (32) et présentant une extrémité distale et une extrémité proximale, les dites mâchoires (30) étant enfilables sur le guide (20) avec leur extrémité proximale tournée vers chacune des extrémités du guide;
- un mandrin (34) enfilable sur les deux extrémités du guide (20) et apte à rapprocher l'un de l'autre les axes des deux mâchoires (30);

2. Clamp chirurgical suivant la revendication 1 **caractérisé en ce qu'**une gaine intermédiaire (28) est mise en place à l'aide du guide (20).

3. Clamp chirurgical suivant l'une quelconque des revendications précédentes **caractérisé en ce que** les mâchoires (30) présentent une section malléable (33) du côté de leur extrémité proximale.

4. Clamp chirurgical suivant l'une quelconque des revendications précédentes **caractérisé en ce que** le mandrin (34) comprend un seul canal longitudinal (37), l'extrémité distale de ce canal (32) étant profilée de façon à entraîner le rapprochement des axes des deux mâchoires (30) par un déplacement relatif.

5. Clamp chirurgical suivant l'une quelconque des revendications 1 à 3 **caractérisé en ce que** le mandrin (34) comprend deux canaux longitudinaux (37), chacune des deux mâchoires (30) étant enfilées dans un des deux canaux (37).

6. Clamp chirurgical suivant l'une quelconque des revendications précédentes **caractérisé en ce que** les mâchoires (30) sont pourvues près de leur extrémité distale de mors souples (36).

7. Clamp chirurgical suivant l'une quelconque des revendications précédentes **caractérisé en ce que** les mâchoires (30) sont serrées par un dispositif de solidarisation à articulation unique ou multiples.

8. Clamp chirurgical suivant l'une quelconque des revendications précédentes **caractérisé en ce que** la fermeture des mâchoires (30) est assurée par une tige de commande (35) enfilée sur les deux extrémités du guide (20) en amont du mandrin (34).

9. Clamp chirurgical suivant l'une quelconque des revendications précédentes **caractérisé en ce que** la partie proximale des mâchoires (30) est munie de cannelures coopérant avec un pas de vis ménagé à la surface interne du mandrin (34).
